# EUROPEAN PATENT APPLICATION

(11) **EP 4 745 222 A1**
(43) Date of publication of application: **20.05.2026**
(21) Application number: 24212835.3
(22) Date of filing: 13.11.2024
(51) Int. Cl.: C12M 1/107, C12M 1/34

(54) **FULLY FLOODED VERTICAL LOOP BIOREACTOR SYSTEM, BIOREACTOR AND CONTINUOUS METHOD FOR AEROBIC GAS-SUBSTRATE FERMENTATION**

(71) Applicant: Aerbio A/S, 2600 Glostrup (DK)
(72) Inventor: Mansfield, Robert, 2600 Glostrup (DK); Mulligan, Katie, 2600 Glostrup (DK); Creer, Peter, 2600 Glostrup (DK)
(74) Representative: Willnegger, Eva

(57) **Abstract**

The present invention relates to the field of aerobic gas-substrate fermentation, in particular for Cupriavidus through a fully flooded vertical loop reactor system.

The present invention further relates to a fully flooded vertical loop bioreactor (100) for aerobic gas-substrate fermentation.

The present invention further relates to a method for aerobic gas-substrate fermentation using the fully flooded vertical loop bioreactor (100) of the invention.

## Description

### FIELD OF THE INVENTION

The present invention relates to the field of aerobic gas-substrate fermentation, using hydrogen oxidizing bacteria, in particular for Cupriavidus sp., through a fully flooded vertical loop reactor system.

The present invention further relates to a fully flooded vertical loop reactor for aerobic gas-substrate fermentation.

The present invention further relates to a method for aerobic gas-substrate fermentation in a fully flooded vertical loop reactor.

### BACKGROUND

For autotrophic Cupriavidus fermentation, aerobic mixtures of H2 and CO2 (or alternate C1 carbon source: e.g. methanol, formate, carbon monoxide) are required. The combining of flammable ratios of O2 with a fuel gas such as H2 presents a risk of explosion. Explosions can present a significant safety risk where the quantities of fuel-oxygen mixtures are sufficiently high that the energy generated upon ignition can not be safely contained, quenched, or otherwise managed. Explosions occurring within confined spaces such as the interior of a bioreactor or the associated pipework can be particularly hazardous, and so it is critical that a robust and effective basis of safety is implemented in the design and operation of such systems. Suitable basis of safety for mixing flammable gasses generally includes one or more of the following:
1) Limiting the quantity of flammable gas mixtures
2) Preventing the formation of oxygen-fuel gas mixtures which fall within the flammable range
3) Providing adequate inerting or quenching of possible energy releases
4) Removal of ignition sources

Within the design constraints of an effective basis of safety, large-scale commercial aerobic gas fermentation requires that systems be designed and operated for high production rates and desirable substrate conversion-yields.

The low solubility of substrate gasses in the fermentation broth requires adding an excess amount of both O2 and H2 at high partial pressures to support high production rates. This poses a challenge for limiting the quantity of potential flammable gas mixtures within the system at a given time.

Gasses are added into fermentation broths where they can be consumed by the fermenting microbes. Unconsumed and/or evolved gasses will often coalesce and collect at high points in the systems, often described as head-spaces, or in de-gasing chambers. Such collecting of gasses presents a risk of formation of a flammable atmosphere if the composition of the gas mixture is not effectively managed. Submerged bubbles of flammable gas mixtures can in principle also represent an explosion risk.

In order for submerged bubbles to present a material safety risk the propagation of a single bubble explosion must be capable of passing to neighboring bubbles and initiating a chain reaction. Bubble to bubble explosion propagation requires an appropriate balance of bubble-gas-composition, bubble-size, localized spatial distribution of bubbles, liquid quenching effect (i.e. gas-liquid ratio / hold-up).

Fermentation metabolism can be influenced by a wide range of factors and so flammable mixtures of O2 and H2 cannot easily be ruled out through control of the input substrate gasses alone without relying on the addition of significant volumes of inerting gasses.

Adding inerting gasses to the broth reduces productivity due to limiting the partial pressure of the substrate gasses. Adding significant volumes of inerting gasses to a headspaces/de-gassing chambers/similar is costly and can result in the necessitate additional complexity and expense for downstream gas separation requirements.

EP4138568A1 to Deep Branch Biotechnology discloses a bioreactor for Cupriavidus fermentation with a feedback loop control of oxygen input, hydrogen input, and carbon dioxide input based on through-put analysis. Inorganic nitrogen is added based on pH and OD-based feedback loops. Oxygen preferably remains at less than 5% in the headspace of the system. However, this bioreactor requires a degassing space and, thus, is not fully flooded.

US10538730 to Calysta shows a loop reactor with nonvertical pressure reduction zones. However, this loop reactor requires a degassing or head tank during standard operating conditions and, thus, it is not fully flooded.

Hence, there remains a need to provide improved systems, reactors and methods for aerobic gas-substrate fermentation, in particular by chemoautotrophic microorganisms such as Cupriavidus with increased efficacy and reduced risk associated with the accumulation of flammable gasses.

### SHORT DESCRIPTION OF THE INVENTION

The inventors surprisingly found that maintaining the gas holdup in vertical loop bioreactor for continuous aerobic bacterial gas-substrate fermentation, in particular of Cupriavidus, in a specific range combined with maintaining a dispersed flow of the multi-phase fermentation mixture comprising gas substrate bubbles, bacteria and liquid media reduces the risk of the buildup and of the explosion of flammable gas mixtures such as a mixture of gaseous hydrogen and oxygen.

Specifically, it was found that by using a specially configured fully-flooded bioreactor system it is possible to create and maintain safe non-explosion-propogating conditions even while simultaneously adding substrate gasses with flammable concentration ratios. This can be achieved without the requirement for inerting gas inputs. Such substrate gas concentration ratios can support higher mass transfer and therefore higher production rates.

In one embodiment, ignition propagation from a first gas bubble to a second gas bubble is thereby reduced. No head tank or degassing space is required or formed during standard operating conditions.

In one embodiment, the superficial velocity within the vertical loop bioreactor, in particular the top horizontal section (30) and the vertical downflow section (20), is maintained at around 0.5 m/s to 2.5 m/s. The maintenance of the superficial velocity in the upper horizontal section avoids the creation of hazardous environments and reduces the loss of system gas.

In one embodiment, the gas volume fraction ("GVF" or "holdup") is controlled at 5 % to 20 % (v/v) as measured by ultrasound or sonar as compared to the total volume of the vertical loop bioreactor.

Gaseous hydrogen, oxygen, carbon dioxide are supplied to the vertical loop bioreactor (100) to generate gas bubbles of between 100 microns to 500 microns diameter as measured by Acoustic Bubble Spectrometry and/or Laser Diffraction.

The loss of gas and the explosion risk is reduced as compared to systems which use a degassing space during standard operating conditions.

Accordingly, a first aspect of the invention is a vertical loop bioreactor (100) for continuous aerobic bacterial gas-substrate fermentation in particular of Cupriavidus, comprising
▪ a vertical upflow section (10) having a diameter d1 in fluid connection with a vertical downflow section (20);
▪ a vertical downflow section (20) having a diameter d2 in fluid connection with the vertical upflow section (10);
▪ a top horizontal section (30) having a diameter d3, a top point (31) and enabling a fluid connection between the vertical upflow section and vertical downflow section; wherein the top horizontal section preferably is a V-shaped to enable the escape of the gas fraction in case of a system failure;
▪ a bottom horizontal section (40) having diameter d4, low point (41) and enabling a fluid connection between the vertical upflow section (10) and vertical downflow section (20); wherein the top horizontal section preferably is a V-shaped to enable the escape of the gas fraction in case of pump failure;
▪ a circulation means (50), preferably one or more than one pumps, even more preferably one or more than one twin-screw pumps or open impeller centrifugal pumps, preferably arranged in the bottom horizontal section (40), even more preferably arranged at the low point (41) of the bottom horizontal section (40);
▪ optionally one or more means to control the bubble size in the horizontal sections (30, 40) and vertical sections (10, 20) to obtain a homogenous distribution of the bubbles in the multi-phase fermentation mixture and to prevent bubble coalescence into hazardous environments; preferably the means to control bubble size are static mixers (210), preferably the static mixers (210) at entry points of the upper and downer loop sections or column;
▪ optionally to prevent coalescence at the high points of the horizontal section barreling to induce swirl flow in the broth, preferably barreling achieved by installation of interior baffles within the horizontal section;
▪ optionally cooling means maintaining a temperature of multi-phase fermentation mixture of between 25°C and 42°C, preferably of between 32 °C and 37°C;
▪ one or more feedback-controlled gas inputs, preferably a hydrogen gas input (61), a carbon dioxide input (62), an oxygen input (63) and nitrogen input (66, 67);
▪ one or more feedback-controlled liquid media inputs, preferably a recycle centrate input (64) and a fresh media input (65);
▪ one or more feedback-controlled outlets for removing multi-phase fermentation mixture from the fully flooded vertical loop bioreactor (100) and further processing of the removed fermentation broth in a degassing vessel (70) optionally connected to a vent (71) and a harvesting area (72);
▪ optionally a deluge tank (80) in fluid connection with the top horizontal section (30), comprising a liquid space (81) and a gas space (82), wherein the liquid space (81) preferably is an aqueous solution and even more preferably is water; and wherein the volume of the liquid preferably is controlled to correspond to the gas volume fraction ("GVF"); and wherein in case of failure of the circulation pump (50), the liquid space (81) is configured to enter the vertical loop and the gas in the vertical loop escapes to the head tank thereby preventing a gas buildup;
   Wherein the vertical loop bioreactor (100) is fully flooded with a multi-phase fermentation mixture comprising gas bubbles, microorganisms, and liquid media;
   Preferably wherein the creation of a hazardous environment through flammable gases is prevented.

In another aspect, the gas bubbles are homogeneously distributed in the multi-phase fermentation mixture.

In another aspect, the median diameter of the bubbles in the multi-phase fermentation mixture is 6000 microns or less, preferably 2000 microns or less, even more preferably 1000 microns or less, even more preferably 500 microns or less, even more preferably 250 microns or less, and even more preferably is between 100 microns and 250 microns as measured by Acoustic Bubble Spectrometry and/or Laser Diffraction.

In another aspect, the gas volume fraction in the multi-phase fermentation mixture is 35 % or less, preferably 30 % or less, and even more preferably between 5 and 20 % (v/v) as compared to the total inner volume of the vertical loop bioreactor (100).

In another aspect, a superficial velocity as measured by Coriolis flowmeter is maintained in the top horizontal section (30) and/or the vertical downflow section (20) sufficient to maintain a gas phase, to prevent the disengagement of bubbles and formation of a hazardous environments in the vertical loop bioreactor (100).

In another aspect, the superficial velocity in the top horizontal section (30) and/or the vertical downflow section (20) is maintained at 0.25 m/s tp 5 m/s, preferably 0.5 m/s to 3.0 m/s, and more preferably, 0.75 m/s to 2.0 m/s.

In another aspect, the vertical downflow section is wider in diameter than one or both horizontal sections to support slower superficial velocities and longer bubble residence time within the section.

In another aspect, the flow rate and/or GVF along the horizontal and/or vertical downflow section is measured, preferably by non-invasive method, and more preferably by ultrasound or sonar-based flow measurement technology fitted externally to the sections.

In another aspect, the lower horizontal section (40) is in a V-shape and wherein the circulation means (50) is located at the low point (41) of the V-shape so as to allow bubbles to migrate to the deluge tank in case of failure of the circulation means (50).

In another aspect, the circulation means (50) is selected to avoid an accumulation of flammable gas into a localized hazardous environment and preferably is a twin screw pump.

In another aspect, the top section (82) of deluge tank (80) is filled with an inert gas and preferably is continuously purged with an inert gas, preferably, wherein the inert gas is nitrogen or carbon dioxide.

In another aspect, the vertical loop bioreactor (100) does not contain a degassing headspace under normal operating conditions.

In another aspect, the vertical downflow section (20) is a tank or a tube dimensioned in function of the superficial velocity.

In another aspect, the vertical downflow section (20) preferably is a tank to allow for active fermentation.

In another aspect, gas bubbles are purged into the multi-phase fermentation mixture with a median bubble diameter of 100 microns to 250 microns so as to avoid an immediate rise to the top of the vertical upflow and downflow sections (10, 20).

In another aspect, the bubble size and the superficial velocity of the mixed-phase mixture is measured by ultrasound or sonar.

In another aspect, the loss of substrate gases is 5 % or less, preferably 2,5 % or less, even more preferably 1 % or less (v/v) as compared to the input substrate gas.

A further aspect of the present invention is a method for continuous aerobic bacterial gas-substrate fermentation in particular of Cupriavidus, comprising:
▪ providing the vertical loop bioreactor (100) of the invention;
▪ passing a multi-phase fermentation mixture comprising a gaseous phase and a liquid phase through the fully flooded vertical loop bioreactor (100);
▪ introducing microorganisms, preferably bacteria, even more preferably Cupriavidus, even more preferably Cupriavidus necator, nutrients, water, and at least one gaseous substrate into the multi-phase fermentation mixture;
▪ maintaining a dispersed flow of the multi-phase fermentation mixture by passing the multi-phase fermentation mixture through a circulation means (50) with a superficial velocity in the top horizontal section (30) and optionally the vertical downflow section (20) of up to 10 m/s, preferably of from 0.75 m/s to 2.0 m/s;
▪ optionally further dispersing the gaseous phase in the multi-phase fermentation mixture by one or more mixing means, preferably a static mixer (210);
▪ maintaining the gas volume fraction of from 0 to 30%, preferably of from 5 % to 25%, even more preferably or of from 5 % to 20 %, even more preferably from 10 % to 15 % (v/v) and even more preferably around 12,5 % as compared to the total inner volume of the vertical loop bioreactor (100),

Wherein the vertical loop bioreactor (100) is fully flooded with a multi-phase fermentation mixture comprising gas bubbles, microorganisms, and liquid media;

Preferably wherein the creation of a hazardous environment through flammable gases is prevented.

Another aspect of the present invention is a system for continuous aerobic bacterial gas-substrate fermentation in particular of Cupriavidus, comprising the vertical loop bioreactor (100) of the invention or configured to carry out the method for continuous aerobic bacterial gas-substrate fermentation.

### DETAILED DESCRIPTION OF THE INVENTION

The invention is now described in further detail.

### Aerobic gas-substrate fermentation

Providing a gas phase (injection median bubble size 100-2000 microns) comprising a carbon-containing molecule, an oxygen-containing molecule, a bioavailable nitrogen-containing molecule and hydrogen, with a maximum median diameter of 6000 microns, preferably less than 2000 microns, preferably less than 1000 microns, even more preferably less than 500 microns, preferably less than 250 microns as measured by ultrasound or sonar.

### Vertical loop bioreactor (100)

In one embodiment, the fully flooded vertical loop bioreactor of the invention comprises:
▪ a vertical upflow section (10) having a diameter d1 in fluid connection with a vertical downflow section
▪ a vertical downflow section (20) having a diameter d2 in fluid connection with the vertical upflow section
▪ a top horizontal section (30) having a diameter d3, a top point (31) and enabling a fluid connection between the vertical upflow section and vertical downflow section; wherein the top horizontal preferably is a V-shaped to enable the escape of the gas fraction in case of pump failure;
▪ a bottom horizontal section (40) having diameter d4, low point (41) and enabling a fluid connection between the vertical upflow section and vertical downflow section; wherein the top horizontal preferably is a V-shaped to enable the escape of the gas fraction in case of pump failure;
▪ a circulation means (50), preferably a pump, even more preferably one or more than one twin-screw pump or open impeller centrifugal pump, preferably arranged in the bottom horizontal section;
▪ a hydrogen gas input (61);
▪ a carbon dioxide input (62);
▪ oxygen input (63);
▪ recycle centrate input (64);
▪ fresh media input (65);
▪ nitrogen input (66), (67);
▪ a degassing vessel (70) optionally connected to a vent (71);
▪ a harvesting means (72);
▪ optionally a deluge tank (80) in fluid connection with the top horizontal section, also referred to as "head tank", comprising a liquid space (91) and a gas space (92), wherein the liquid preferably is an aqueous solution and even more preferably is water; and wherein the volume of the liquid is preferably is controlled to correspond to the Gas volume fraction ("GVF") or "holdup"; and wherein in case of circulation pump failure, the liquid is configured to enter the vertical loop and the gas buildup escapes to the head tank;

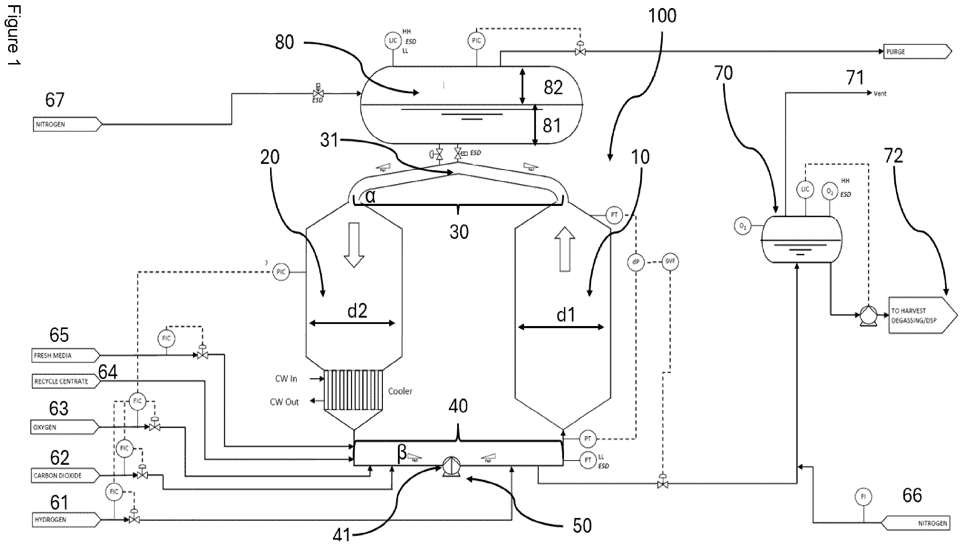

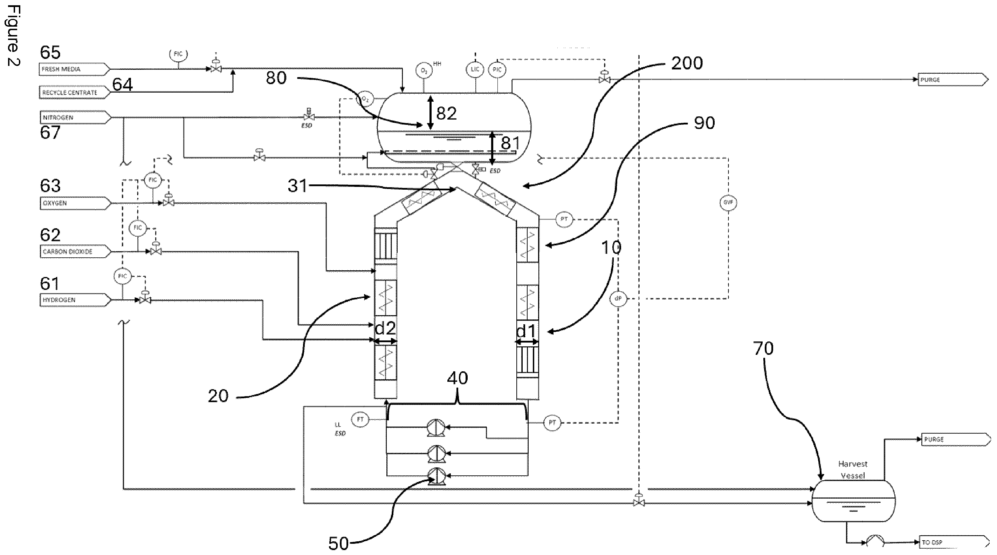

Preferred vertical loop bioreactors of the invention are shown in Figures 1 and 2.

In one embodiment, top and bottom means against or in gravity direction.

Upflow and downflow are used herein in line with gravity. Downflow means in gravity direction. Topflow means in opposite direction of gravity.

In one embodiment, the reactor has an internal volume to accommodate a multi-phase fermentation mixture comprising a liquid phase and gaseous phase, of from 2 to 1000 m3, of from 5 to 1000 m3, of from 5 to 800 m3, of from 5 to 600 m3, 2 to 500 m3, more preferably of from 5 to 500 m3, of from 5 to 400 m3, of from 5 to 300 m3, of from 5 to 200 m3, of from 5 to 100 m3, of from 10 to 500 m3, of from 10 to 400 m3, of from 10 to 300 m3, of from 10 to 200 m3, of from 10 to 100 m3, of from 20 to 500 m3, of from 20 to 400 m3, of from 20 to 300 m3, of from 20 to 200 m3, of from 20 to 100 m3, of from 50 to 500 m3, of from 50 to 400 m3, of from 50 to 300 m3, of from 50 to 200 m3, or of from 50 to 100 m3.

In one embodiment, the reactor operates as follows:
▪ A multi-phase fermentation mixture comprising a gaseous phase and a liquid phase is circulated in the loop reactor by the circulation means (50). The multi-phase fermentation mixture comprises growing and fermenting microorganisms, for example hydrogen-oxidizing bacteria. The multi-phase fermentation mixture circulates in the direction from the circulation means vertical upflow section (10) to the top horizontal section (30) and then further to vertical downflow section (20) and then to bottom horizontal section (40) back to the downstream loop part.
▪ A liquid stream comprising water and nutrients or pH-adjusting compounds is intermittently introduced into the multi-phase fermentation mixture via an inlet configured for receiving a liquid stream.
▪ A gaseous substrate inlet configured for introducing a gaseous substrate into a liquid introduces oxygen, hydrogen, and carbon dioxide gaseous substrates into the multi-phase fermentation mixture.
▪ A gas holdup of up to 30 %, but preferably between 5 % to 20 % (v/v) and a dispersed flow are maintained in a way that no hazardous environment, and in particular no headspace is generated in the reactor and the reactor remains fully flooded.
▪ Bacterial biomass and liquid exits the reactor or continuous pipe at intervals via a liquid outlet;
▪ Continuous monitoring and adjustment of process conditions results in an equilibrium of gaseous substrate fluxes to avoid the formation of a hazardous environment, such as a headspace.

### Multi-phase fermentation mixture - fermentation broth

The fully flooded vertical loop bioreactor comprises a multi-phase fermentation mixture comprising a one or more gas phases, bacteria and a liquid phase in which the one or more gas phases and the bacteria are homogenously distributed.

The terms "multi-phase fermentation mixture" and "fermentation broth" are used interchangeably.

### Continuous process

The fully flooded vertical loop bioreactor is configured to enable a continuous process for the production of biomass.

In one embodiment, the fully flooded vertical loop bioreactor comprises a continuous pipe. Continuous means that the loop reactor or pipe is a continuous structure suitable throughout for fermentation. Elements do not interrupt the vertical loop bioreactor or continuous pipe with sections of equal or varying diameter, in particular:
▪ an inlet configured for receiving a liquid stream,
▪ a liquid outlet zone comprising a liquid outlet,
▪ one or more gaseous substrate inlets configured for introducing a gaseous substrate into a liquid, and
▪ a mixing means such as a sieve, grate, or baffle, configured to maintain a dispersed flow or configured to disperse the gaseous substrate into the liquid.

### Fully flooded system and absence of a degassing headspace under operating conditions

The vertical loop bioreactor (100) is maintained fully flooded during standard operating conditions. Accordingly, biomass extracted from the vertical loop reactor system and media input streams are controlled to maintain a fully flooded system.

In one embodiment, fully flooded means that no hazardous environments are present in the vertical loop bioreactor in operating mode.

Examples of hazardous environments are large gas bubbles containing a gas volume equivalent to spherical bubbles of diameter greater than 2 cm or greater than 4 cm or greater than 8 cm containing flammable gas mixtures or localized accumulations of flammable gas compositions with less than 80% liquid, or less than 50% liquid, or less than 20% liquid, or less than 10% liquid, or less than 5% liquid, or less than 2.5% liquid, or less than 1% liquid, or less than 0.2% liquid in the inner volume of the vertical loop bioreactor (100) of the vertical upflow section (20), specifically wherein the potential pressure release from the ignition of an accumulation of such flammable gas volume exceeds the capacity of the system to dissipate the energy without exceeding the design pressure of the vessel. Another example of hazardous environment is where uneven Gas Volume Fraction leads to the formation of a local distribution of bubbles which when combined with bubble volumes in that area contains sufficient energy potential for propagation of a chain reaction of ignitions with neighboring bubbles, which occurs by intersection or by compression when neighboring bubbles reach Auto Ignition Temperatures (AIT). AIT can be calculated as AIT as a function of pressure and equivalence ratio. Gas Volume Fraction can be measured by sonar based measurement of the mixed-phase broth or gravimetric methods or by measuring the ingoing and outgoing compositions of gaseous and liquid and mixed-phase flows. Bubble distribution can be measured by Conductivity wire-mesh sensor. To determine the safe operating envelope, the potential risk of propagation of bubble-to-bubble explosions within the system can be calculated using the mass of the flammable gas mixture and the GVF and the AIT. The AIT for the hydrogen-oxygen mixture can be calculated as a function of pressure (p) and equivalence ratio (ϕ). The equivalence ratio (ϕ) represents the actual hydrogen-to-oxygen ratio in the system relative to the stoichiometric ratio for hydrogen-oxygen combustion (2:1). In this context, scalar parameters are coefficients that adjust the AIT calculation to account for specific effects of pressure and mixture composition on ignition characteristics in hydrogen-oxygen systems. If the release of energy from a single bubble to the system exceeds the AIT for neighboring bubbles when taking into account the heating of the aqueous boundary layer around the bubble and energy dissipation of combustion heat then the capacity for explosion propagation can be assumed.

The fully flooded vertical loop bioreactor does not require a degassing headspace under normal operating conditions.

Only in case of a system failure or slowdown, for example if the circulation means (50), preferably a twin screw pump, stops working or works in a reduced way.

In one embodiment, the deluge tank is operated if the superficial velocity in the horizontal or downflow sections falls below 2,5 m/s, preferably below 1,5 m/s, preferably below 1 m/s, even more preferably below 0,5 m/s.

Preferably, no gas/liquid separation unit is present in the vertical loop bioreactor or continuous pipe according to the invention.

In one embodiment, during fermentation no excess gas or headspace is generated in the continuous fully flooded vertical loop bioreactor or continuous pipe.

Input substrates and/or microorganism growth rates are controlled to reduce headspace generation through excess gas. Gas produced by the microorganisms is subsequently consumed by the microorganism present in the fully flooded vertical loop reactor or continuous loop according to the invention.

### Gas volume fraction ("GVF") or gas holdup

The term "gas hold-up" is defined as the volume fraction of gas in the liquid phase in the bioreactor comprising the input gases and any other gases formed in the liquid phase.

In one embodiment, the input stream preferably comprises maintaining a molar ratio of hydrogen: oxygen gas hold-up in the liquid phase of from 0.5 : 1 to 7 : 1.

The gas volume fraction ("GVF") is preferably kept at less than 35 %, preferably less than 30 %, even more preferably between 2 % and 20 % (v/v), even more preferably between 5 % and 15 % (v/v) as compared to the total volume of the horizontal top and bottom sections and the vertical downflow and vertical upflow section of the vertical loop bioreactor.

The gas volume fraction ("GVF") is preferably kept maintained between 5 % and 20 % (v/v).

Preferably, the cross-sectional bubble distribution is even, wherein even means symmetric or homogenous distribution of the bubbles throughout the system:
The gas is injected for example by spargers to generate a median gas bubble size of preferably between 10 and 2000 micron diameter, preferably between 20 and 1000 micron diameter, preferably between 50 and 500 micron diameter, and more preferably between 100 and 250 micron diameter as measured by Acoustic Bubble Spectrometry and/or Laser Diffraction.

The inventors have found that maintaining the gas holdup in a specific range combined with maintaining a dispersed flow of the multi-phase fermentation mixture reduces the risk of explosion of flammable gas mixtures such as a mixture of gaseous hydrogen and oxygen. In particular, the risk of ignition propagation from a first gas bubble to a second gas bubble is greatly reduced.

A minimum gas holdup is required for sufficient mass-transfer of the gaseous substrate to the growing microorganisms or bacteria in the bioreactor. Too high levels of gas holdup increase the risk of forming explosive gas bubbles. Explosive gas bubbles at high enough gas holdups can propagate explosions.

The gas holdup is preferably maintained at a substantially constant level, preferably by matching the input of each gaseous substrate with its consumption in the system. Therefore, gas holdup needs to be continuously monitored or determined. Various means of determining gas holdup are known in the art and can be located at different positions in or on the system depending on their mode of operation.

Preferably, the gaseous phase comprises bubbles with a diameter of less than 50 mm, preferably less than 20 mm, more preferably less than 10 mm, 6 mm, 2 mm, 1 mm or 0.5 mm, in the multi-phase fermentation mixture. Maintaining a small bubble size prevents the formation of explosive gas bubbles and/or the risk of propagation of explosive gas bubbles.

### Minimization of loss of substrate gases

A loss of substrate gases of less than 5 %, less than 2,5 % or less than 1 % as compared to the input substrate gas.

### Superficial velocity and flow rate

Maintaining a superficial velocity in the horizontal and/or downflow section configured to:
▪ To maintain a gas phase,
▪ to prevent the disengagement and formation of a hazardous environment in the system
▪ wherein the 0,25 m/s to 5 m/s, 0.5 m/s to 3.0 m/s, even more preferably 0.75 m/s to 2.0 m/s in the top section and a similar range in the vertical downflow section;
wherein the system is fully flooded system.

### Reduction of the build-up of a hazardous environment through flammable gas accumulation

The maintenance of a superficial velocity of the invention and the homogeneous distribution of the gas phase in the multi-phase fermentation mixtures prevents the creation of hazardous environment. Hazardous environment means zones filled with flammable gas in an amount sufficient to propagate an explosion.

### Substrate gases and ratios

In one embodiment, hydrogen is added to the mixed-phase liquid in a concentration of from 70 % to 100 % (v/v), oxygen in a concentration of from 20 % to 100 % (v/v) and carbon dioxide in a concentration of from 5 % to 100 % (v/v) to the liquid phase, individually or as any premixed combination thereof.

In one embodiment, substrate gases are substrate gases are added to the mixed-phase liquid at a molar ratio of hydrogen : oxygen : carbon dioxide of from 3.88 to 51.94 : 0.85 to 2 : 0.75 to 2 to the liquid phase.

In a preferred embodiment, substrate gases are added to the mixed-phase liquid at a molar ratio of dissolved hydrogen : oxygen : carbon dioxide of from 3.183 to 12.748 : 0.795 to 4.25 : 0.75 to 2.0 in the liquid phase.

In one embodiment, the molar ratio of hydrogen : oxygen gas hold-up in the liquid phase of from 0.5 : 1 to 7 : 1, preferably of from 1 : 1 to 3.5 : 1.

### Vertical upflow section (10)

The fully flooded vertical loop bioreactor comprises at least one top horizontal section (30) having a diameter d3 enabling a fluid connection between the vertical upflow section and at least one vertical downflow section (20).

In a preferred embodiment, the top horizontal preferably is a V-shaped to enable the escape of the gas fraction in case of pump failure.

### Flow direction

The flow direction of the mixed-phase mixture in the fully flooded vertical loop bioreactor (100) may be reversed.

### Vertical downflow section (20)

The fully flooded vertical loop bioreactor comprises a vertical downflow section (20) having a diameter d2. The vertical downflow section (20) is in fluid communication with the vertical upflow section (10).

The vertical downflow section (20) may be a tank or a pipe. In one embodiment, a tank means a diameter (d2) larger than the diameter top horizontal section (d3).

Preferably, the vertical downflow section (20) is a tank to allow for active fermentation.

In one embodiment, the vertical downflow section (20) is a pipe, i.e. a diameter (d2) equal than the diameter top horizontal section (d3). In this embodiment, the vertical downflow section (20) preferably comprises one or more static mixers. This embodiment is shown in Figure 2.

### Top horizontal section (30) with high point (31)

The fully flooded vertical loop bioreactor (100) comprises a top horizontal section (30) having a diameter d3 enabling a fluid connection between the vertical upflow section and vertical downflow section. The top horizontal preferably is V-shaped to enable the escape of the gas fraction in case of pump failure;

### Bottom horizontal section (40) with low point (41)

The fully flooded vertical loop bioreactor (100) comprises a bottom horizontal section (40) having diameter d4 enabling a fluid connection between the vertical upflow section and vertical downflow section.

Preferably, the top horizontal is a V-shaped having a low point (41) to enable the escape of the gas fraction in case of pump failure.

### V-shape to enable gas escape (31, 41)

Key performance characteristics of the twin screw pump located at the bottom of a V-shape (so that bubbles migrate to the deluge tank in case the twin screw pump failure, i.e. the superficial velocity is not sufficient to entrain gas, deluge tank connection close to the high tank) or other pumps with similar functions: selected to avoid vertexing (accumulation of gas into a localized hazardous environment (big bubble).

In one embodiment, V-shaped means an inclination along their longitudinal axis with a respective angle α from 0.1 to 20 degrees relative to the horizontal plane.

In one embodiment, V-shaped means inclined along their longitudinal axis with a respective angle β from 0.1 to 20 degrees relative to the horizontal plane.

### Circulation means (50)

The fully flooded vertical loop bioreactor (100) comprises at least one circulation means (50) enabling the continuous circulation of the mixed phase li. Preferably, the circulation means (50) is a pump, preferably a twin screw pump.

Even more preferably, the fully flooded vertical loop bioreactor (100) comprises a single circulation means, preferably a twin screw pump (50) and no further circulation means, such as further pumps.

The circulation means (50) is configured to circulate the multi-phase fermentation mixture within the fully flooded vertical loop bioreactor (100). Preferably the circulation (50) means is positioned at or near the lowest point of the fully flooded vertical loop bioreactor (100).

### Static mixers (90)

In one embodiment, the fully flooded vertical loop bioreactor (100) comprises one or more means (90) to break up bubbles in horizontal, vertical sections to obtain an even or homogenous distribution of the gas phase. The breakup means (90) is configured to maintain a bubble size of the gas phase of 2 cm or less, preferably of 1 cm or less, even more preferably of

In a preferred embodiment, the breakup means (90) are static mixers.

In one embodiment, static mixers (90) are arranged at the entry points of the upper and downer loop sections or column.

### Deluge tank (80)

The fully flooded vertical loop bioreactor (100) preferably comprises a deluge tank comprising a liquid space (81) and a head space (82) with a liquid volume of at least the volume of the gas volume fraction (GVF) in the fully flooded vertical loop bioreactor (100).

In a preferred embodiment, the headspace (82) of the deluge tank (80) is filled with inert gas.

### Bubble generation and maintenance

The fully flooded vertical loop bioreactor (100) comprises bubble generation means (69) for dosing the gaseous substrate into the fully flooded vertical loop reactor (100). The bubble generation means (69) preferably are nozzles.

The bubble generation means preferably are generating bubbles in the size of 250 microns or less.

### Measurement method for gas phase and bubbles

Ultrasound/sonic as non-invasive measurement method for superficial velocity.

### System temperature

In one embodiment, the temperature of the multi-phase fermentation mixture is held at between 25 °C and 42 °C, preferably between 32 °C to 37 °C through temperature regulation means, such as a heat exchanger.

### Substrate/nutrient inlet flows (61, 62, 63, 65, 65, 66, 67) - Multi-phase fermentation mixture

The multi-phase fermentation mixture comprises bacteria, dissolved and suspended ingredients, liquid media and homogeneously distributed gas bubbles, collectively referred as the gas phase of the multi-phase fermentation mixture.

Multi-phase fermentation mixture and fermentation broth are used interchangeably.

Gas inlet flows are controlled to maintain a fixed gas hold-up volume. The ratio control on the individual gas inlet flows ensures that the gas feed stoichiometry is maintained.

The gas hold-up volume does not exceed 30 % (v/v) and preferably is between 5 to 20 % (v/v) as compared to the total inner volume of the fully flooded vertical loop bioreactor system (100).

In one embodiment, the gas volume fraction is measured using vessel level and differential pressure:
▪ An increase in gas volume fraction above the operating range may affect the circulation pump performance and capacity, thereby reducing the superficial liquid velocity and increasing the risk of flammable gases building up into; and
▪ A decrease in gas volume fraction affects the mass transfer rate.

Nutrients comprise carbon - and/or nitrogen - and/or phosphorous-comprising compounds. Nutrients are present in the fully flooded vertical loop bioreactor (100) as part of the multi-phase fermentation mixture. Substrate/nutrient inlet flows are feedback loop controlled.

Preferable the nutrients comprise:
▪ carbon-comprising compound, preferably carbon dioxide,
▪ nitrogen-comprising-compounds, preferably ammonia,
▪ optionally phosphorous-comprising compounds.

Gaseous substrates, such as hydrogen, oxygen and carbon dioxide are injected directly into the reactor (100) by way of a gas generation system and spargers configured to generate gas bubbles preferably of between 100 and 250 microns diameter.

Fresh liquid growth media is preferably provided in a preparation tank comprising salt solutions metered into water, preferably purified water, and fed to the fully flooded vertical loop bioreactor (100) preferably by dosing pumps.

NH3 is supplied to the fully flooded vertical loop bioreactor (100) preferably via a dosing pump in the form of an aqueous ammonia solution.

Ammonia addition is controlled to keep the pH in the fully flooded vertical loop bioreactor (100) stable. pH correcting agents, such as acid and base may be added to stabilize the pH.

Antifoam agents may be added to prevent the generation of foam within the fully flooded vertical loop bioreactor (100).

In a preferred embodiment, recycled media, also referred to as "centrate", is extracted from the harvested fermentation broth and then fed back into the fully flooded vertical loop bioreactor (100).

Maintaining nutrient concentrations sufficient for maintaining a physiological steady state lies within the ambit of the skilled person. In this steady state, growth occurs at a constant specific growth rate and all culture parameters remain substantially constant (culture volume, dissolved oxygen concentration, nutrient and product concentrations, pH, cell density, etc.).

(Gaseous) substrate inlet (61, 62, 63, 65, 65, 66, 67) are configured for introducing a gaseous substrate into a liquid and preferably are positioned downstream of, upstream of, or next to, the circulation means (50).

**Substrate** inlet flows (61, 62, 63, 65, 65, 66, 67) are controlled to maintain a maximum pressure of between 0 and 8 barg, or more preferably between 1 and 4 barg in the fully flooded vertical loop bioreactor (100).

In one embodiment, the introduction and/or replacement speed of the influences controls the specific growth rate of the microorganisms. Increasing the dilution rate will increase the growth of the microorganisms.

However, the dilution rate needs to be controlled relative to the specific growth rate to prevent wash-out. The dilution rate is preferably controlled in order to maximize the production of the biological product, or the protein production rate and protein content of the microorganisms. When the specific growth rate of the microorganisms is too high the protein content and/or quality of the biomass can be reduced.

In one embodiment, the liquid phase of the multi-phase fermentation mixture in the fully flooded vertical loop bioreactor (100) is replaced at a rate of from 4 to 30 % per hour, preferably of from 8 to 20 % per hour; and/or maintaining a concentration of the bacteria in the liquid phase of the fully flooded vertical loop bioreactor (100) of at least 10 g/l, preferably of from 10 to 100 g/l.

### Gas addition control through feedback loop

The addition of substrate gases is controlled through feedback loops to ensure a gas holdup of 5 % to 20 % (v/v) as compared to the total inner volume of the vertical loop bioreactor (100).

### Harvest controlled to maintain maximum gas holdup of 20%

In one embodiment, the fermentation broth is harvested from a side-stream from the circulation line and transferred to a harvest degassing vessel (70) before transferring to a harvest buffer storage vessel prior to downstream processing (72).

In one embodiment, the harvest outlet flow is controlled to ensure maximum gas holdup of 20 % (v) as compared to the total inner volume of fully flooded vertical loop bioreactor (100).

Impurities within input gas streams can build up over time within the fully flooded system. In one embodiment, the fermentation broth degassing vessel (70) facilitates the removal of non-substrate gas impurities prior to recycling the liquid back to the fully flooded system.

Typically, the harvest separation vessel is operated at a reduced pressure as compared to the fully flooded vertical loop bioreactor (100).

Typically, unreacted dissolved gas will come out of solution along with entrained gases and be vented from the harvest degassing vessel under backpressure control.

In one embodiment, the degassing process (70) will be supplemented with nitrogen stripping.

The system is designed to enable a high biomass production rate. The high production rate is preferably measured by dry weight measurement of the biomass harvested.

The system is designed to enable high production rates of greater than 0.25 g/L/h, or more preferably more than 0.5 g/L/h, or more preferably more than 1.0 g/L/h, or more preferably more than 1.5 g/L/h, or more preferably more than 2.0 g/L/h, or more preferably more than 2.5 g/L/h, or more preferably more than 3.0 g/L/h, or more preferably more than 3.5 g/L/h, or more preferably more than 4.0 g/L/h.

Where a L is 1000 cubic centimeters of internal reactor volume and a g is a gram of biomass and/or other carbon containing metabolic product of fermentation.

The system is designed to enable a high working biomass concentration, which supports lower dewatering costs in downstream processing of the bioreactor harvest streams. The high working biomass is preferably measured by dry weight measurement of samples of the bioreactor content.

The system is designed to maintain a high working biomass concentration of greater than 5 g/L, or more preferably more than 10 g/L, or more preferably more than 15 g/L, or more preferably more than 20 g/L, preferably more than 30 g/L.

The system is designed to enable a biomass product of high protein content. Preferably more than 60% protein, more preferably more than 65% protein, even more preferably more than 70% protein, even more preferably more than 75% protein, even more preferably more than 80% protein, even more preferably more than 85% protein, even more preferably more than 90% protein.

Protein content is determined by nitrogen analysis using the Dumas method and/or by calculating the sum of amino acid concentrations.

The system enables control of the fermentation such that favorable metabolic conversion yields of the substrate inputs are achieved. The efficiency of metabolic conversion yield is preferably measured by mass balance across the system, where input gas quantities are measured by mass flow meter, and biomass product within the harvest stream is measured by gravimetric dry mass measurement, and any unused gas is measured by mass flow meter and mass spectrometer.

The measured molar ratio of carbon fixed to hydrogen utilized in the formation of 1 C-mol of biomass is preferably less than 1:10, more preferably less than 1:7, more preferably less than 1:6.5, more preferably less than 1:6, more preferably less than 1:5.5, more preferably less than 1:5, more preferably less than 1:4.5, more preferably less than 1:4.

The system specifically enables that while maintaining a safe operating envelope it is possible to simultaneously achieve a high production rate, and the production of a high protein biomass product, and a favorable metabolic conversion yield of substrates into product, and minimal losses of substrate gasses from the system.

### Biomass amino acid and lipid fingerprint

In one embodiment, the biomass obtainable by the method, the system and the bioreactor of the invention comprises an amino acid content comprising a histidine content of from 1.2 to 3.2 % of total biomass dry weight protein content, an isoleucine content of from 2.6 to 4.6 % of total biomass dry weight protein content, a leucine content of from 5.0 to 8.0 % of total biomass dry weight protein content, a lysine content of from 4.0 to 7.4 % of total biomass dry weight protein content, a methionine content of from 1.4 to 3.6 % of total biomass dry weight protein content, a phenylalanine content of from 2.3 to 5.7 % of total biomass dry weight protein content, a threonine content of from 2.1 to 4.6 % of total biomass dry weight protein content, a tryptophan content of from 0.5 to 2.6 % of total biomass dry weight protein content, and a valine content of from 2.2 to 6.2 % of total biomass dry weight protein content.

In one embodiment, the biomass obtainable by the method, the system and the bioreactor of the invention comprises a lipid content of from 2.3 to 18 % of total biomass dry weight comprising a fatty acid content comprising a C16:0 palmitic acid content of from 23 to 60 % of total biomass dry weight fatty acid content, a C16:1 palmitoleic acid content of from 3.8 to 22.3 % of total biomass dry weight fatty acid content, and a C17:1 heptadecenoic acid content of from 23 to 60 % of total biomass dry weight fatty acid content.

### Use

In one embodiment, the biomass obtainable by the method, the system and the bioreactor of the invention is used to feed or provide nutrition to one or more organisms, preferably wherein the organisms comprise fish, crustaceans, molluscs, poultry, pigs and cattle.

In another embodiment, the biomass obtainable by the method, the system and the bioreactor of the invention is used as a food, a food ingredient or a functional food.

### Further applications

In one embodiment, the method, the system and the bioreactor of the invention are used to produce alcohols, solvents, acids, purified protein, cell extracts, amino acids, biopolymers and, vitamins and nutrients, pharmaceutical compounds.

Examples of Biopolymers are polyhydroxyalkanoates.

Examples of acids include lactic acid, levulinic acid, formic acid, acetic acid, 2, 5-furandicarboxylic acid (FDCA), and sugar acids, butyric acids.

### Microorganisms

Preferred microorganisms are hydrogen-oxidizing bacteria capable of reproducing on a multi-gas mixture of oxygen gas, hydrogen gas, carbon dioxide gas, carbon monoxide gas, and methane gas.

Examples include *Rhodopseudomonas* sp.; *Rhodospirillum* sp.; *Rhodococcus* sp.; *Rhodobacter* sp.; *Rhizobium* sp.; *Thiocapsa* sp.; *Pseudomonas* sp.; *Nocardia* sp.; *Hydrogenomas* sp.; *Hydrogenobacter* sp.; *Hydrogenovibrio* sp.; *Helicobacter* sp.; *Xanthobacter* sp.; *Hydrogenophaga* sp.; *Bradyrhizobium* sp.; *Ralstonia* sp.; *Gordonia* sp.; *Mycobacteria* sp.; *Alcaligenes* sp.; *Cupriavidus* sp.; *Variovorax* sp.; *Acidovorax* sp.; *Anabaena* sp.; *Scenedesmus* sp.; *Chlamydomonas* sp.; *Ankistrodesmus* sp.; *Rhaphidium* sp. or *Arthrobacter* sp., and combinations thereof.

A particularly preferred microorganism is Cupriavidus sp, and even more preferably Cupriavidus necator.

In one embodiment, the fermentation broth comprises a cocktail of microorganisms.

In one embodiment, the specific growth rate of from 1.0 to 8.0 d⁻¹ or of from 0.04 to 0.3 h⁻¹.

### Nitrogen sources

In one embodiment, ammonium hydroxide and/or other bioavailable nitrogen sources are supplied to the process, directly and/or through incorporation into liquid media input stream and/or recycled liquid streams, such that a minimum of 10 g of atomic nitrogen is provided for every 100 g of biomass produced, controlled as part of a feedback loop in response to the density of the cells in the liquid phase and dilution rate. In addition to the gaseous substrates, hydrogen-oxidizing bacteria require a source of bioavailable nitrogen for production of proteins.

This nitrogen is a major contributing factor to protein content, independent of respective molecular associations such as in ammonium hydroxide (NH4OH) or ammonium chloride (NH3Cl). There is no maximum threshold foreseen as excess bioavailable nitrogen in the liquid phase will be recycled.

In one embodiment, 5 g to 5000 g of atomic nitrogen is provided for every 100 g of biomass produced.

### Gas phase, ratio, controlled input and output streams

The gaseous phase is understood herein to mean a volume consisting of gaseous material and in contact with a liquid phase.

The gaseous phase in a bioreactor is commonly called the headspace and is typically located directly above the liquid phase.

To clarify, gas or gaseous substrate sparged into the liquid phase is not the gaseous phase but becomes part of the gaseous phase upon exiting the liquid phase.

In one embodiment, the new input gases and/or recycled input gases are added to the bioreactor contain non-substrate gas volumes of less than 90 % substrate gas, more preferably less than 60 %, more preferably less than 30 %, more preferably less than 10 %, more preferably less than 5 %, more preferably less than 1 %, even more preferably less than 0.1 % (v/v) of the total input gas volume.

Gaseous substrate is understood to mean a gaseous supply of nutrients and/or energy for growth and/or maintenance of microorganisms.

Cement kiln is understood to mean a space used for the pyro-processing stage of manufacture of Portland and other types of hydraulic cement, in which calcium carbonate reacts with silica-bearing minerals to form a mixture of calcium silicates.

Syngas, or synthesis gas, is herein understood to mean a mixture comprising carbon monoxide, carbon dioxide and hydrogen. Syngas is produced by gasification of a carbon containing fuel to a gaseous product.

The exact chemical composition of syngas varies based on the raw materials and the processes. One of the uses of syngas is as a fuel to manufacture steam or electricity.

Another use is as a basic chemical building block for many petrochemical and refining processes. Syngas can be produced from many sources, including natural gas, coal, petroleum-based materials, bio-mass, other materials that would be rejected as waste, or virtually any hydrocarbon feedstock.

*"Knallgas"* is understood to mean a mixture of hydrogen and oxygen gases, which is highly combustible. A molar ratio of 2 : 1 is sufficient for maximum ignition efficiency.

In the present method, oxygen and hydrogen gas inputs are controlled as part of a feedback loop to allow for initial addition of the gas to the system above the explosive limit, as it is injected initially into the liquid media phase.

The feedback control then ensures that oxygen and/or hydrogen is being sufficiently utilised by the system to ensure that no hazardous environment is created.

Examples include gas accumulation resulting in a headspace above explosive safety limits, of 5 % (v/v) for oxygen and 4 % (v/v) for hydrogen as compared to the inner volume of the bioreactor under standard conditions. Additionally, all three gas inputs are controlled such that oxygen or hydrogen is maintained as the limiting gas within the system.

Sparging is understood to mean a process in which a gas is bubbled through a liquid.

Combining gas controls with defined parameters for background conditions, media compositions, inorganic nitrogen addition, and dilution rate, advantageously enables modulation of biomass composition through the direct metabolic and physiological limitations imposed on the microbes.

Ammonium hydroxide and/or other bioavailable nitrogen sources are supplied to the process, directly and/or through incorporation into liquid media input stream and/or recycled liquid streams, such that a minimum of 10 g of atomic nitrogen is provided for every 100 g of biomass produced, controlled as part of a feedback loop in response to the density of the cells in the liquid phase and dilution rate.

In addition to the gaseous substrates, hydrogen-oxidizing bacteria require a source of bioavailable nitrogen for production of proteins.

This nitrogen is a major contributing factor to protein content, independent of respective molecular associations such as in ammonium hydroxide (NH4OH) or ammonium chloride (NH3Cl). There is no maximum threshold foreseen as excess bioavailable nitrogen in the liquid phase will be recycled.

In one embodiment, the input stream is controlled comprising adding a molar ratio of hydrogen: oxygen to the liquid phase of from 1 : 1 to 10 : 1.

In one embodiment, the input stream is controlled comprising maintaining a molar ratio of hydrogen: oxygen gas hold-up in the liquid phase of from 1 : 1 to 3.5 : 1.

In one embodiment, the input stream is controlled comprising maintaining a concentration of hydrogen of from 0.5 to 20 mg/l, a concentration of oxygen of from 0.5 to 80 mg/l and a concentration of carbon dioxide of from 20 to 2000 mg/l in the liquid phase at a temperature of from 28 to 45 °C and at a pressure in the gaseous phase of from 100 to 2000 kPa.

In one embodiment, the input stream is controlled comprising adding hydrogen, oxygen and carbon dioxide in a molar ratio of hydrogen : oxygen : carbon dioxide of from 2 to 6 : 0.85 to 2 : 0.75 to 2 to the liquid phase.

In one embodiment, the input stream is controlled comprising maintaining a molar ratio of dissolved hydrogen : oxygen : carbon dioxide of from 3.183 to 12.748 : 0.795 to 4.25 : 0.75 to 2.0 in the liquid phase.

In one embodiment, the input stream controlling the input stream comprises maintaining a concentration of hydrogen of from 0.5 to 20 mg/l, a concentration of oxygen of from 0.5 to 80 mg/l and a concentration of carbon dioxide of from 20 to 2000 mg/l in the liquid phase at a temperature of from 28 to 45 °C and at a pressure in the gaseous phase of from 100 to 2000 kPa.

In one embodiment, the input stream and nutrient composition is controlled comprising maintaining a specific growth rate of the microorganisms of from 1.0 to 8.0 d⁻¹, preferably from 3.0 to 5.5 d⁻¹, even more preferably from 3.5 to 5.o d⁻¹ or from 0.04 to 0.3 h⁻¹.

In one embodiment, the input stream and nutrient composition is controlled comprising maintaining a concentration of the microorganisms in the liquid phase of the bioreactor of from 10 to 100 g/l.

In one embodiment, the input stream is controlled comprising maintaining hydrogen at a transfer rate of from 0.03 to 1.2 mol/l/h in the liquid phase and/or oxygen at a transfer rate of from 0.003 to 0.4 mol/l/h in the liquid phase;

In one embodiment, the input stream is controlled comprising maintaining a molar ratio of hydrogen : oxygen gas hold-up in the liquid phase of from 0.5 : 1 to 7 : 1,

In one embodiment, the input stream and nutrient composition is controlled comprising maintaining a concentration of the microorganisms in the liquid phase of the bioreactor of at least 10 g/l, and

In one embodiment, the input and outlet streams are controlled comprising replacing and/or recycling the liquid phase of the bioreactor where the microorganisms are grown and maintained at an hourly volume of from 4 % to 30 %, preferably from 5 % to 20 %, more preferably from 10 % to 20 %, alternatively or from 20 % to 30 % (v/v) as compared to the inner volume of the bioreactor.

In one embodiment, the gas composition in the bioreactor or any of the inputs or output streams is measured directly or indirectly by mass spectrometry or laser-based methods such as laser diffraction (volume by volume ratio quantification) preferably combined with a mass or volume measurement such as a muss flow meter.

### Gas recycling

In a preferred embodiment, unused gas substrates removed from the system via the harvest stream may optionally be recycled to the bioreactor for further fermentation.

### Liquid media

The liquid phase may comprise carbon, nitrogen and/or phosphorous-comprising compounds, wherein carbon-comprising compounds may be formate or methanol, but preferably are understood to be essentially limited to dissolved CO2, or urea, wherein the latter may also be considered a bioavailable N2 source.

Formate or methanol can be converted to CO2 in the liquid phase of the bioreactor, which can for example be catalyzed by enzymes present inside or outside microorganisms, thereby indirectly acting as a supply of a gaseous substrate.

In one embodiment, the liquid media input is controlled comprising adding a nutrient composition which is adjusted to a pH of from 1 to 4 prior to adding.

In one embodiment, the nutrient composition comprises DSMZ medium for chemolithotrophic growth 81 (H-3) without NaHCO3 and wherein preferably prior to adding the medium is supplemented with an additional 1.5 g/L ammonium chloride, 3 × 10⁻⁴ g/L NiCl2 × 6H2O, and 1.5 × 10⁻³ g/L ZnSO4 × 7H2O, and 1.5 × 10⁻⁴ g/L CuCl2 × 2H2O, and 0.15 g/L Ferric ammonium citrate.

### Liquid media recycling

In a preferred embodiment, unused liquid media substrates removed from the system via the harvest stream, such as the centrate, may optionally be recycled to the bioreactor for further fermentation.

### pH

The liquid stream may also comprise a pH-adjustment liquid steam, preferably comprising a suitable base such as NaOH or NH4OH. The pH-adjustment stream is introduced in the liquid phase in order to maintain the pH of the liquid phase at a pH which is physiologically suitable for the microorganisms, preferably the bacteria, in the system according to the invention. Preferably, the physiologically suitable pH is of from 6 to 7.5, of from 6.5 to 8, or of from 6 to 8, more preferably the pH is of from 6.5 to 7 or of from 6.5 to 7.5.

### Temperature and pressure

In a preferred embodiment, the temperature of the fully flooded vertical loop bioreactor (100) is held at 35 °C and the top pressure is kept at between 0 and 4 barg and more preferably between 1.0 and 2.0 barg.

The fermentation process is exothermic. Therefore, in one embodiment cooling means, such as heat exchange, are arranged around the vertical upflow section (10) and/or the vertical downflow section (20) of the fully flooded vertical loop bioreactor (100).

### Liquid outlet and harvest

The fully flooded vertical loop bioreactor (100) comprises an outlet zone comprising one or more outlet pipes for continuously removing fermentation broth.

In one embodiment, the diameter of the liquid outlet pipe is 5 % to 100 %, or more preferably 10 % - 40 % of the diameter of the horizontal section of the fully flooded vertical loop bioreactor (30).

In one embodiment, the diameter of the liquid outlet pipe is 1 % - 100 %, or more preferably 2 % - 50 %, or more preferably 3 % - 25 %, or more preferably 5 % - 20 % of the diameter of the top horizontal section (30) or the bottom section (40) of the fully flooded vertical loop bioreactor (100).

### Purging of inert gases from the fermentation vessel

In one embodiment, inert gases are purged from the fermentation vessel via the harvest degassing vessel (70). The degassing vessel (70) will remove entrained and dissolved gas from the harvest treat optionally followed by nitrogen stripping.

### Upstream processing

In one embodiment, the gaseous substrate is subjected to a pre-processing step.

The pre-processing may include purifying gas, in particular carbon dioxide, oxygen or hydrogen derived from the exhaust gas of a production or combustion process or other gas production process.

In one embodiment, the input gas stream is purified to obtain a volume consisting of essentially only substrate gases such as carbon dioxide wherein other elements of the exhaust gas are substantially removed by devices and methods known in the art such as for example by using electrostatic precipitators or baghouses to remove ash and other particulates, using denitrification units to remove nitrogen oxides, using wet scrubbers, spray-dry scrubbers or dry sorbent injection systems to remove sulfur oxides.

Carbon dioxide can be captured in post-combustion processes by separation methods known in the art, for example by using a solvent such as an amine to form a carbonate salt.

The carbon dioxide is absorbed by the solvent after which it can be released by heat to form a highly purified stream of carbon dioxide.

### Downstream processing

Dry weight or dry matter of a material herein is understood to mean the material consisting of all its constituents, but essentially excluding water. Examples of obtaining the dry weight or dry matter of a material are using centrifugation, drum drying, belt drying, evaporation, freeze drying, heating, spray drying, vacuum drying and/or vacuum filtration such that the water content of the material is removed.

In one embodiment, the harvested biomass may be further subject to downstream processing.

Downstream processing is understood to mean one or more processing steps applied to a liquid phase removed from a bioreactor, which may include a kill-step process, a dewatering process, or a drying process.

Kill-step is understood to mean a process that achieves the reproductive inactivation of microorganisms. This process may occur within the liquid phase, a dewatered liquid phase, or dried biomass, for example by using an ultra-highpressure homogenizer, acid, base, solvent, or heat-based microbial killing methods.

Dewatering is understood herein to mean a first process of removing liquid and/or a nutrient composition from biomass or a composition comprising biomass. Examples of dewatering are centrifugation, evaporation, heating, tangential flow filtration, and vacuum filtration. Dewatering may be followed by further downstream processing.

Drying is understood to mean a process of removing water from biomass or a composition comprising biomass to produce a biomass consisting of all its constituents, but essentially excluding water.

Examples of drying are drum drying, belt drying, freeze drying, spray drying and vacuum drying.

### Measurement methods and means

In one embodiment, the fully flooded vertical loop bioreactor (100) comprises means for determining one or more of:
▪ liquid level,
▪ gas holdup,
▪ gaseous substrate concentration,
▪ bubble diameter, more preferably gas holdup, and
▪ liquid flow.

In one embodiment, the fully flooded vertical loop bioreactor (100) comprises one or more of
▪ a tomography sensor configured to detect electrical impedance, neutron radiation, sound waves, or electromagnetic radiation or waves, such as X-rays;
▪ a density sensor;
▪ a conductivity sensor;
▪ a wire mesh sensor;
▪ a load cell;
▪ a visual inspection means;
▪ an optical sensor, such as a high speed camera;
▪ an ultrasound sensor; and
a differential pressure sensor.

Preferably the measurement means are non-invasive, such as ultrasound or sonar.

In a particularly preferred embodiment, the measurement method is ultrasound.

### Method

A further aspect of the present invention is a method for producing biomass or a biological product in the fully flooded vertical loop bioreactor (100), comprising:
▪ passing a multi-phase fermentation mixture comprising a gaseous phase and a liquid phase through the fully flooded vertical loop bioreactor (100);
▪ introducing microorganisms, nutrients, water, and at least one gaseous substrate into the multi-phase fermentation mixture;
▪ maintaining a dispersed flow of the multi-phase fermentation mixture by passing the gaseous phase through a circulation means (50) at to maintain a superficial velocity in the top horizontal section (30) and optionally the vertical downflow section (20) of up to 60 m/s, preferably of up to 10 m/s, preferably of from 1.5 m/s to 2.5 m/s, and by passing the liquid phase at a speed of at least 1.5 m/s, preferably of at least 4 m/s, or preferably of from 1.5 m/s to 2.5 m/s;
▪ optionally further dispersing the gaseous phase in the multi-phase fermentation mixture by one or more mixing means, preferably a static mixer;
▪ maintaining the gas holdup of from 0 % to 30%, preferably of from 5 % to 25% or of from 10 % to 15%, more preferably of about 12.5%.

### SHORT DESCRIPTION OF THE DRAWINGS

Figure 1 shows a preferred embodiment of the fully flooded vertical loop bioreactor (100) wherein the both the vertical upflow section (10) and the vertical downflow sections (20) are in the form of tanks characterized by a diameter d1 and d2 larger than the diameter of the top horizontal section and the bottom horizontal section (d3, d4).
Figure 2 shows an alternative embodiment of the fully flooded vertical loop bioreactor (200) wherein the both the vertical upflow section (10) and the vertical downflow sections (20) are in the form of pipes characterized by a diameter d1 and d2 equal to the diameter of the top horizontal section (230) and the bottom horizontal section (40), (d3, d4). In this alternative embodiment, static mixers (90) are employed to ensure a homogeneous distribution of the bubbles within the multi-phase fermentation mixture.

### DETAILEED DESCRIPTION OF THE DRAWINGS

### Figure 1

Figure 1 shows an exemplary embodiment of the fully flooded vertical loop bioreactor (100) of the invention comprising:
▪ A vertical upflow section (10) having a diameter d1 in fluid connection with a vertical downflow section;
▪ A vertical downflow section (20) having a diameter d2 in fluid connection with the vertical upflow section;
▪ A top horizontal section (30) having a diameter d3, a top point (31) and enabling a fluid connection between the vertical upflow section and vertical downflow section; wherein the top horizontal preferably is a V-shaped to enable the escape of the gas fraction in case of pump failure;
▪ A bottom horizontal section (40) having diameter d4, low point (41) and enabling a fluid connection between the vertical upflow section and vertical downflow section; wherein the top horizontal preferably is a V-shaped to enable the escape of the gas fraction in case of pump failure;
▪ a circulation means (50), preferably a pump, even more preferably a twin screw pump or open impeller centrifugal pumps, preferably arranged in the bottom horizontal section;
▪ a hydrogen gas input (61);
▪ a carbon dioxide input (62);
▪ oxygen input (63);
▪ recycle centrate input (64);
▪ fresh media input (65);
▪ nitrogen input (66), (67;)
▪ a degassing vessel (70) optionally connected to a vent (71);
▪ a harvesting means (72);
▪ a deluge tank (80) in fluid connection with the top horizontal section, also referred to as "head tank", comprising a liquid space (81) and a gas space (82), wherein the liquid preferably is an aqueous solution and even more preferably is water; and wherein the volume of the liquid is preferably is controlled to correspond to the Gas volume fraction ("GVF") or "holdup"; and wherein in case of circulation pump failure, the liquid is configured to enter the vertical loop and the gas buildup escapes to the head tank.

A feedback loop control of oxygen/air input (63), hydrogen input (61), and carbon dioxide input (62) based on through-gas analysis and/or analysis of their concentrations in the fully flooded vertical loop bioreactor (100) is controlled through a defined and adjustable input gas ratio for optimal protein production metabolism.

pH and OD based feedback loops are present for inorganic nitrogen (e.g. urea or ammonia) addition (66, 67) per unit biomass produced.

The pH is maintained through addition of a pH buffer. Liquid growth media (64, 65) is added to the fully flooded vertical loop bioreactor (100) as needed in response to growth of the microorganisms measured through the sensors of the various feedback loops. Unutilized gas may be recycled into the bioreactor.

The dilution rate is controlled to allow for a predetermined maintenance time in the reactor and determined by controlling the inputs of inorganic nitrogen (66, 67), optional pH buffer, fresh and/or recycled liquid growth media (64, 65) and the removal of liquid containing biomass (70, 80).

Removal of liquid containing biomass is followed by a downstream processing step involving dewatering in which a large part of the liquid is separated from the liquid containing biomass. The large part of the liquid is then preferably recycled (64) to the fully flooded vertical loop bioreactor (100).

Biomass together with a small part of the remaining liquid is optionally subjected to additional downstream processing steps (9) comprising further dewatering, drying and inactivation of the microorganisms. Responsive oxygen feeding (63) is preferred to improve reactor load while remaining within safe gas mixture concentrations within the fully flooded vertical loop bioreactor (100). Ammonia is also fed in response to defined process conditions growth rates.

### Figure 2

Figure 2 shows an exemplary embodiment of the fully flooded vertical loop bioreactor (200) of the invention comprising:
▪ A vertical upflow section (10) having a diameter d1 in fluid connection with a vertical downflow section;
▪ A vertical downflow section (20) having a diameter d2 in fluid connection with the vertical upflow section;
▪ A top horizontal section (30) having a diameter d3, a top point (31) and enabling a fluid connection between the vertical upflow section and vertical downflow section; wherein the top horizontal preferably is a V-shaped to enable the escape of the gas fraction in case of pump failure;
▪ A bottom horizontal section (40) having diameter d4, low point (41) and enabling a fluid connection between the vertical upflow section and vertical downflow section; wherein the top horizontal preferably is a V-shaped to enable the escape of the gas fraction in case of pump failure;
▪ Static mixers (90);
▪ a circulation means (50), preferably a pump, even more preferably a twin screw pump, preferably arranged in the bottom horizontal section;
▪ a hydrogen gas input (61);
▪ a carbon dioxide input (62;)
▪ oxygen input (63);
▪ recycle centrate input (64);
▪ fresh media input (65);
▪ nitrogen input (66);
▪ a degassing vessel (70) optionally connected to a vent (71);
▪ a harvesting means (72);
▪ a deluge tank (80) in fluid connection with the top horizontal section, also referred to as "head tank", comprising a liquid space (81) and a gas space (82), wherein the liquid preferably is an aqueous solution and even more preferably is water; and wherein the volume of the liquid is preferably is controlled to correspond to the Gas volume fraction ("GVF") or "holdup"; and wherein in case of circulation pump failure, the liquid is configured to enter the vertical loop and the gas buildup escapes to the head tank.

A feedback loop control of oxygen/air input (63), hydrogen input (61), and carbon dioxide input (62) based on through-gas analysis and/or analysis of their concentrations in the fully flooded vertical loop bioreactor (200) is controlled through a defined and adjustable input gas ratio for optimal protein production metabolism.

pH and OD based feedback loops are present for inorganic nitrogen (e.g. urea or ammonia) addition (66, 67) per unit biomass produced.

The pH is maintained through addition of a pH buffer. Liquid growth media (64, 65) is added to the fully flooded vertical loop bioreactor (200) as needed in response to growth of the microorganisms measured through the sensors of the various feedback loops.

Unutilized gas may be recycled into the bioreactor. The dilution rate is controlled to allow for a predetermined maintenance time in the reactor and determined by controlling the inputs of inorganic nitrogen (66, 67), optional pH buffer, fresh and/or recycled liquid growth media (64, 65) and the removal of liquid containing biomass (70, 80).

Removal of liquid containing biomass is followed by a downstream processing step involving dewatering in which a large part of the liquid is separated from the liquid containing biomass.

The large part of the liquid is then preferably recycled (64) to the fully flooded vertical loop bioreactor (200). Biomass together with a small part of the remaining liquid is optionally subjected to additional downstream processing steps comprising further dewatering, drying and inactivation of the microorganisms.

Responsive oxygen feeding (63) is preferred to improve reactor load while remaining within safe gas mixture concentrations within the fully flooded vertical loop bioreactor (200). Ammonia is also fed in response to defined process conditions growth rates.

## Claims

1. A vertical loop bioreactor (100) for continuous aerobic bacterial gas-substrate fermentation of hydrogen oxidizing bacteria, in particular of Cupriavidus, comprising
▪ a vertical upflow section (10) having a diameter d1 in fluid connection with a vertical downflow section (20);
▪ a vertical downflow section (20) having a diameter d2 in fluid connection with the vertical upflow section (10);
▪ a top horizontal section (30) having a diameter d3, a top point (31) and enabling a fluid connection between the vertical upflow section and vertical downflow section; wherein the top horizontal section preferably is a V-shaped to enable the escape of the gas fraction in case of a system failure;
▪ a bottom horizontal section (40) having diameter d4, low point (41) and enabling a fluid connection between the vertical upflow section (10) and vertical downflow section (20); wherein the top horizontal section preferably is a V-shaped to enable the escape of the gas fraction in case of pump failure;
▪ a circulation means (50), preferably one ore more pumps, even more preferably one or more twin screw pumps or open impeller centrifugal pumps, preferably arranged in the bottom horizontal section (40), even more preferably arranged at the low point (41) of the bottom horizontal section (40);
▪ optionally one or more means to control the bubble size in the horizontal sections (30, 40) and vertical sections (10, 20) to obtain a homogenous distribution of the bubbles in the multi-phase fermentation mixture and to prevent bubble coalescence into hazardous environments; preferably the means to control bubble size are static mixers (210), preferably the static mixers (210) at entry points of the upper and downer loop sections or column;
▪ optionally cooling means maintaining a temperature of multi-phase fermentation mixture of between 25 °C and 42 °C, preferably of between 32 °C and 37 °C;
▪ one or more feedback-controlled gas inputs, preferably a hydrogen gas input (61), a carbon dioxide input (62), an oxygen input (63) and nitrogen input (66, 67);
▪ one or more feedback-controlled liquid media inputs, preferably a recycle centrate input (64) and a fresh media input (65);
▪ one or more feedback-controlled outlets for removing multi-phase fermentation mixture from the fully flooded vertical loop bioreactor (100) and further processing of the removed fermentation broth in a degassing vessel (70) optionally connected to a vent (71) and a harvesting area (72);
▪ optionally a deluge tank (80) in fluid connection with the top horizontal section (30), comprising a liquid space (81) and a gas space (82), wherein the liquid space (81) preferably is an aqueous solution and even more preferably is water; and wherein the volume of the liquid preferably is controlled to correspond to the gas volume fraction ("GVF"); and wherein in case of failure of the circulation pump (50), the liquid space (81) is configured to enter the vertical loop and the gas in the vertical loop escapes to the head tank thereby preventing a gas buildup.
Wherein the vertical loop bioreactor (100) is fully flooded with a multi-phase fermentation mixture comprising gas bubbles, microorganisms, and liquid media;
Preferably wherein the creation of a hazardous environment through flammable gases is prevented.

2. The vertical loop bioreactor (100) according to claim 1, wherein the gas bubbles are homogeneously distributed in the multi-phase fermentation mixture.

3. The vertical loop bioreactor (100) according to any one of the preceding claims, wherein the median diameter of the bubbles in the multi-phase fermentation mixture is 6000 microns or less, preferably 2000 microns or less, even more preferably 1000 microns or less, even more preferably 500 microns or less, even more preferably 250 microns or less and even more preferably is between 100 microns and 250 microns as measured by acoustic bubble spectrometry and/or laser diffraction.

4. The vertical loop bioreactor (100) according to any one of the preceding claims, wherein the gas volume fraction in the multi-phase fermentation mixture is 35 % or less, preferably 30 % or less, and even more preferably between 5 % and 20 %(v/v) as compared to the total inner volume of the vertical loop bioreactor (100).

5. The vertical loop bioreactor (100) according to any one of the preceding claims, wherein a superficial velocity is maintained in the top horizontal section (30) and/or the vertical downflow section (20) sufficient to maintain a gas phase, to prevent the disengagement of bubbles and formation of a hazardous environments in the vertical loop bioreactor (100);
Preferably, wherein superficial velocity in the top horizontal section (30) and/or the vertical downflow section (20) is maintained at 0.25 m/s to 5 m/s, preferably 0.5 m/s to 3.0 m/s, and more preferably, 0.75 m/s to 2.0 m/s.

6. The vertical loop bioreactor (100) according to any one of the preceding claims, wherein the lower horizontal section (40) is in a V-shape and wherein the circulation means (50) is located at the low point (41) of the V-shape so as to allow bubbles to migrate to the deluge tank in case of failure of the circulation means (50).

7. The vertical loop bioreactor (100) according to any one of the preceding claims, wherein the circulation means is selected to avoid an accumulation of flammable gas into a localized harzardous environment and preferably is a twin screw pump.

8. The vertical loop bioreactor (100) according to any one of the preceding claims, wherein the top section (82) of deluge tank (80) is filled with an inert gas and preferably is continuously purged with an inert gas, preferably, wherein the inert gas is nitrogen.

9. The vertical loop bioreactor (100) according to any one of the preceding claims, wherein the vertical loop bioreactor (100) does not contain a degassing headspace under normal operating conditions.

10. The vertical loop bioreactor (100) according to any one of the preceding claims, wherein the vertical downflow section (20) is a tank or a tube dimensioned in function of the superficial velocity; wherein the vertical downflow section (20) preferably is a tank to allow for active fermentation.

11. The vertical loop bioreactor (100) according to any one of the preceding claims, wherein gas bubbles are purged into the multi-phase fermentation mixture with a median bubble diameter of 100 microns to 250 microns so as to avoid an immediate rise to the top of the vertical upflow and downflow sections (10, 20).

12. The vertical loop bioreactor (100) according to any one of the preceding claims, wherein the bubble size and the superficial velocity of the mixed-phase mixture is measured by ultrasound or sonar.

13. The vertical loop bioreactor (100) according to any one of the preceding claims, wherein the loss of substrate gases is 5 % or less, preferably 2,5 % or less, even more preferably 1 % or less (v/v) as compared to the input substrate gas.

14. Method for continuous aerobic bacterial gas-substrate fermentation in particular of Cupriavidus, comprising:
▪ providing a vertical loop bioreactor (100) according to any one of the preceding claims;
▪ passing a multi-phase fermentation mixture comprising a gaseous phase and a liquid phase through the fully flooded vertical loop bioreactor (100);
▪ introducing microorganisms, preferably bacteria, even more preferably Cupriavidus, even more preferably Cupriavidus necator, nutrients, water, and at least one gaseous substrate into the multi-phase fermentation mixture;
▪ maintaining a dispersed flow of the multi-phase fermentation mixture by passing the gaseous phase through a circulation means (50) with a superficial velocity in the top horizontal section (30) and optionally the vertical downflow section (20) of up to 10 m/s, preferably of from 0.75 m/s to 2.0 m/s;
▪ optionally further dispersing the gaseous phase in the multi-phase fermentation mixture by one or more mixing means, preferably a static mixer (210);
▪ maintaining the gas volume fraction of from 0 % to 30%, preferably of from 5 % to 25%, even more preferably or of from 5 % to 20 %, even more preferably from 10 % to 15 % (v/v) and even more preferably around 12,5 % as compared to the total inner volume of the vertical loop bioreactor (100),
Wherein the vertical loop bioreactor (100) is fully flooded with the multi-phase fermentation mixture comprising gas bubbles, bacteria and liquid media so as to avoid the creation of a hazardous environment through flammable gases sufficient to propagate an explosion.

15. System for continuous aerobic bacterial gas-substrate fermentation in particular of Cupriavidus, comprising the vertical loop bioreactor (100) of patent claims 1 to 13 or configured to carry out a method according to claim 14.
